# EUROPEAN PATENT APPLICATION

(11) **EP 4 663 745 A1**
(43) Date of publication of application: **17.12.2025**
(21) Application number: 24752630.4
(22) Date of filing: 04.01.2024
(51) Int. Cl.: C12N 1/06, C08B 37/00, C07K 1/34, C07K 1/14, C12N 15/10, A61K 8/99, A61K 8/97, A61Q 19/00

(54) **PRODUCTION PROCESS FOR MICROBIAL CYTOPLASMIC MACROMOLECULAR EXTRACT**

(30) Priority: 09.02.2023 CN 202310088353
(71) Applicant: Beijing Active Blue Crystal Biotechnology Co., Ltd., Beijing 102204 (CN)
(72) Inventor: LING, Feng, Beijing 102204 (CN); DING, Wenyong, Beijing 102204 (CN); SONG, Yanxia, Beijing 102204 (CN)
(74) Representative: Mathys & Squire
(86) International application number: PCT/CN2024/070484
(87) International publication number: WO 2024/164772

(57) **Abstract**

The present invention discloses a process for producing microbial cytoplasm macromolecular extract. The present invention uses microorganisms as raw materials and adopts a low-temperature cell wall breaking method to break the microbial cell wall. The soluble substances in the microbial cytoplasm are released by breaking the cell wall. Then, the solid substances in the cell wall breaking mixture are separated to obtain the soluble substances in the microbial cytoplasm. Then, the medium and small molecular substances containing color, odor and inorganic salts in the soluble substances are removed by microfiltration to obtain concentrated macromolecular substances. The medium and small molecular substances are further removed by repeated dilution with water and then filtration and concentration to obtain a pure colorless, odorless and salt-free cytoplasm macromolecular extract. During the entire production process, a low temperature is always maintained and no other chemical substances and enzymes are added, which ensures that the obtained microbial cytoplasm macromolecular extract maintains its original biological activity.

## Description

### FIELD OF THE INVENTION

The present invention belongs to the field of biotechnology, and more specifically relates to a process of producing microbial cytoplasmic macromolecular extract.

### BACKGROUND OF THE INVENTION

Cytoplasm is the general term for all translucent, colloidal, granular substances surrounded by the cytoplasmic membrane except the nuclear region. The water content is about 80%. The main components of the cytoplasm are ribosomes, storage materials, various enzymes and intermediate metabolites, various nutrients and macromolecular monomers, etc. The cytoplasm includes matrix, organelles and inclusions.

The cytoplasmic matrix, also known as cytosol, is the homogeneous and translucent colloidal part of the cytoplasm, filling between other tangible structures. The cytoplasmic matrix accounts for about 55% of the total volume of the cell, in which there are thousands of enzymes. Most intermediate metabolism (including glycolysis, gluconeogenesis, and the synthesis of sugars, fatty acids, nucleotides and amino acids) is carried out in the cytoplasmic matrix. About 20% of the cytoplasmic matrix is protein. The chemical composition of the cytoplasmic matrix can be divided into three categories according to its relative molecular weight, namely small molecules, medium molecules and macromolecules. Small molecules include water, inorganic ions and small molecule organic monomers, etc.; medium molecules include lipids, oligosaccharides, oligopeptides, nucleotide derivatives, etc.; macromolecules include proteins, fats and lipoproteins, polysaccharides and RNA, etc.

Cell organelles are structures distributed in the cytoplasm, have a certain shape, and play an important role in cell physiological activities.It includes: mitochondria, endoplasmic reticulum, endoplasmic apparatus, Golgi apparatus, lysosomes, microfilaments, microtubules, centrioles, ribosomes, etc. The chemical components mainly include water, proteins and lipids, and also contain a small amount of small molecules such as coenzymes and nucleic acids.

Inclusions are structures in the cytoplasm that have no metabolic activity but have a specific shape. Some are stored energy substances, such as glycogen granules and lipid droplets; some are cell products, such as secretory granules and melanin granules; residual bodies can also be regarded as inclusions.

From the above introduction to the substances contained in the microbial cytoplasm, it can be concluded that the soluble substances in the cytoplasm mainly include proteins, lipids, sugars, nucleic acids and inorganic substances. Proteins include macromolecular proteins, small molecular proteins, polypeptides, oligopeptides and amino acids. Lipids include macromolecular and small molecular fats and lipids, glycerol, fatty acids, etc. Sugars include polysaccharides, oligosaccharides and monosaccharides, etc.

From the above analysis of soluble substances in the cytoplasm, it can be known that the cytoplasm includes macromolecular substances, medium molecular substances and small molecular substances, which have different compositions and different functions. If they can fully play their roles when applied, especially when applied in the field of cosmetics, they should be separated so that they can truly play their due roles. In addition, the medium and small molecular substances in the cytoplasm include color and odor substances and inorganic salts, which will affect the quality of cosmetics, so they must be removed when applied to the field of cosmetics.

Cytoplasmic macromolecules mainly include proteins, fats and lipids, lipoproteins, polysaccharides and RNA and other soluble macromolecules with biological activity. They not only have many benefits for human health, but also have multiple functions in protecting, repairing and treating human skin. The soluble substances in cytoplasmic macromolecules provide skin cells with the necessary macromolecular nutrients with biological activity, such as a large number of enzymes required for growth, which promote the optimal growth state of skin cells and ensure the vigorous metabolism of skin cells, so as to keep the skin healthy. Their main functions in cosmetics are moisturizing, anti-oxidation, inhibiting the production of melanin, whitening, improving skin condition, improving skin roughness, etc.

Therefore, microbial cytoplasm macromolecular extracts play an important role in the application of cosmetics.

During the fermentation and cultivation process of microorganisms, the microbial cytoplasm will contain yellow or yellow-brown substances, substances with special smells and a large amount of inorganic salts. If using traditional cell wall breaking technology, such as autolysis and enzyme hydrolysis, is used, the biological activity of microbial cytoplasm macromolecular substances will also be destroyed.

Color can cause skin pigmentation, and odor can make the produced cosmetics feel unpleasant. Therefore, color and odor can seriously affect the quality of the produced cosmetics. Long-term contact of the skin with inorganic salts can cause irritation to the skin. In particular, excessive salts concentration can cause corrosion, causing skin allergies, erythema, papules, blisters, exudation, itching, and many other symptoms. Once the biological activity of microbial cytoplasmic macromolecules is destroyed during the production process, these substances cannot play their due role, reducing their use effect, thereby affecting the quality of cosmetics. Therefore, if the cytoplasmic macromolecular extract is to be applied to the cosmetic field and exert its maximum effect, the color and odor substances and inorganic salts in the cytoplasmic macromolecular extract must be removed and the biological activity of the substances in the cytoplasmic macromolecular extract must be maintained. The process for producing cytoplasmic macromolecular extract achieved this effect is not mentioned in prior arts. How to solve these problems becomes the key to producing microbial cytoplasm macromolecular extracts suitable for cosmetic applications.

Therefore, it is necessary to find a process for producing microbial cytoplasm macromolecular extract that can achieve decolorization, deodorization, desalination and maintain the biological activity of the substances in the microbial cytoplasm macromolecular extract while retaining the biological activity of the microbial cytoplasm macromolecular extract. This will play a vital role in the application of microbial cytoplasm macromolecular extracts, especially when used as cosmetic raw materials.

### BRIEF DESCRIPTION OF THE DRAWINGS

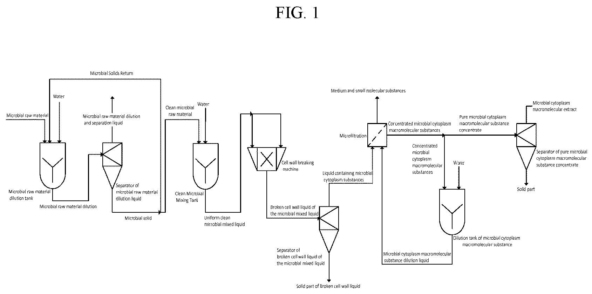

Fig. 1 is a detailed flowchart of the process herein disclosed.

### DETAILED DESCRIPTION

The objective of the present disclosure is to provide a process for producing microbial cytoplasm macromolecular extract that can achieve decolorization, deodorization, desalination and maintain the biological activity of the substances therein while retaining the microbial cytoplasm macromolecular extract.

In various embodiments, the the following technical scheme is disclosed for achieving the objective.

A process of producing microbial cytoplasm macromolecular extract is provided, the process comprising the steps of:
(1) Adding water to a used microbial raw material, preferably mixing evenly, to obtain a microbial raw material dilution, followed by separation of the obtained microbial raw material dilution using a separator, removing the liquid part from the separation, and obtaining a microbial solid; and
(2) Repeating step (1) at least once to obtain a clean microbial raw material; and
(3) Adding water to the clean microbial raw material, preferably mixing evenly, to obtain a uniform clean microbial mixed liquid; and
(4) Breaking the cell wall of the clean microbial mixed liquid to obtain a broken cell wall liquid of the microbial mixed liquid; and
(5) Using a separator to separate the broken cell wall liquid of the microbial mixed liquid, separating and removing the solid part, to obtain a liquid containing microbial cytoplasm substances; and
(6) filtering the obtained liquid containing microbial cytoplasm substances by a microfiltration membrane to remove medium and small molecular substances therein to obtain concentrated microbial cytoplasm macromolecular substances; and
(7) adding water to the concentrated microbial cytoplasm macromolecular substances to obtain a microbial cytoplasm macromolecular substance dilution liquid; and
(8) separating and concentrating the microbial cytoplasm macromolecular substance dilution liquid again by a microfiltration membrane to obtain a first purified microbial cytoplasm macromolecular substance concentrate; and
(9) repeating steps (7) and (8) to remove medium and small molecular substances in the concentrated microbial cytoplasm macromolecular substances to obtain a pure microbial cytoplasm macromolecular substance concentrate; and
(10) separating the obtained pure microbial cytoplasm macromolecular substance concentrate by a high-speed centrifuge to remove solid substances therein to finally obtain a microbial cytoplasm macromolecular extract.

In some aspects, the microorganisms described in step (1) include unicellular eukaryotic microorganisms and/or unicellular prokaryotic microorganisms.

In some aspects, water is added in step (1) to obtain a ratio of water to microorganism not less than about 2 (w/w).

In some aspects, the dry matter content of the microbial solid in step (1) is not less than about 30% (w/w), calculated based on the weight percentage of dry matter contained in the microbial solid.

In some aspects, the separators described in steps (1) and (5) can separate the solid and liquid phases in the separated liquid, and the separators are selected from horizontal screw centrifuges, disc separators, plate and frame filter presses and the like.

In some aspects, repeating step (1), the number of repetitions is not less than 2 times.

In some aspects, adding water to the clean microbial raw material in step (3) ,the amount of water added is 0.5-2 times (w/w) of the microbial raw material.

In some aspects, breaking the cell wall in step (4) comprises a process carried out at low temperature, such as using a homogenizer to break the cell wall.

In some aspects, the medium and small molecular substances in step (6) contain all color and odor substances and inorganic salts in the obtained liquid containing microbial cytoplasm substances.

In some aspects, when adding water to the concentrated microbial cytoplasm macromolecular substances in step (7), the ratio of the amount of water added to the concentrated microbial cytoplasm macromolecular substances is not less than 2 times.

In some aspects, the overall process from step (1) to (10) does comprise addition of enzymes, chemicals, or the like that destroy the biological activity of the substances in the microbial cytoplasm macromolecular extract, and the temperature is maintained at no more than 55° C in the overall process.

In some aspects, the pore size of the microfiltration membrane is 0.05-1 µm.

In some aspects, according to step (9), steps (7) and (8) are repeated for no less than 2 times.

In some aspects, the microbial cytoplasm macromolecular extract in step (10) can be in the form of a concentrated solution as the final productor can be freeze-drying as a lyophilized powder as the final product.

The advantages and beneficial effects of the present invention include:

First, the product produced by the present invention (microbial cytoplasm macromolecular extract) is a brand-new product, and the prior arts do not introduce the same product as the product produced by the present invention.

Second, microbial cytoplasm macromolecular extract include proteins, fats, lipoproteins, polysaccharides, RNA and other soluble macromolecular substances with biological activity. They not only have many benefits for human health, but also have multiple functions for the protection, repair and treatment of human skin. The soluble substances in the cytoplasm macromolecules provide skin cells with macromolecular nutrients with biological activity required for their growth, such as a large number of enzymes required for growth, etc., which promote the optimal growth state of skin cells and ensure the vigorous metabolism of skin cells, so as to keep the skin healthy. Their main functions in cosmetics are moisturizing, anti-oxidation, inhibiting the formation of melanin, whitening, improving skin condition, improving skin roughness and other functions. Therefore, microbial cytoplasm macromolecular extract play an important role in the application of cosmetics.

Third, the present invention adopts steps (2) and (3) to achieve the thorough removal of impurities in the microbial raw materials through multiple dilution and separation of the microbial raw materials, thereby obtaining clean microbial raw materials, laying a good foundation for finally obtaining clean microbial cytoplasm macromolecular extract.

Fourth, the cell wall breaking process described in step (4) is a cell wall breaking process that can be carried out at low temperature, such as using a homogenizer to break the cell wall. The low-temperature cell wall breaking adopted by the present invention will not destroy the biological activity of the substances in the cytoplasm macromolecular extract, thereby ensuring the biological activity of the substances in the produced microbial cytoplasm macromolecular extract, such as the activity of enzymes. Only by ensuring their activity can they fully exert their functions.

Fifth, in the overall process, no enzymes that destroy the biological activity of substances in the microbial cytoplasm macromolecular extract are added. Microbial cytoplasm macromolecular extract containsa large number of biologically active substances, such as enzymes, etc. Only by maintaining their biological activity can they be guaranteed to play their due functions when used. High temperature and the addition of decomposing enzymes and some chemical substances, such as strong acids and strong alkalis, will destroy the biological activity of substances in the microbial cytoplasm macromolecular extract. It can be seen from the process of the present invention that in the entire process, no substances that destroy the biological activity of substances in the microbial cytoplasm macromolecular extract are added, and low temperature is maintained throughout the entire process, thereby ensuring the biological activity of substances in the microbial cytoplasm macromolecular extract.

Sixth, the pore size of the microfiltration membrane described in step (6) is 0.05-1 µm, thereby ensuring the flexibility of the microbial cytoplasm macromolecular extract produced. The size of the microfiltration membrane pore size determines the molecular weight of the microbial cytoplasm macromolecular extract produced by the invention. The smaller the pore size, the smaller the molecular weight of the microbial cytoplasm macromolecular extract produced. According to needs, by adjusting the pore size of the microfiltration membrane, microbial cytoplasm macromolecular extract with different molecular weights can be produced.

Seventh, the present invention completely removes the medium and small molecular substances in the cytoplasm through the method of multiple concentration, dilution and re-concentration in steps (6), (7), (8) and (9). The medium and small molecular substances in the cytoplasm include all color substances, odor substances and inorganic salts in the microbial cytoplasm. The present invention completely removes the color substances, odor substances and inorganic salts through the method of completely removing the medium and small molecular substances in the cytoplasm, thereby obtaining a pure, colorless, odorless, salt-free microbial cytoplasm macromolecular extract that retains the original biological activity.

Eight, the present invention removes solid matter produced in the production process from the produced microbial cytoplasm macromolecular extract through final high separation factor separation, ensuring that the produced microbial cytoplasm macromolecular extract is completely soluble in water.

### EXAMPLES

The present invention will be further described below through specific examples.

### EXAMPLE 1

(1) 600g of water as added to 200g of waste yeast (dry matter content is 33%) which is a byproduct of beer fermentation, the separator was set to 4500 rpm, with a separation factor of 3622, separated for 10 minutes, and the supernatant removed to obtain the yeast solid at the bottom;
(2) 600g of water was added into the yeast solid, stir evenly, the separator set to 4500 rpm, with a separation factor is 3622, separated for 10 minutes, and the supernatant was removed to obtain the yeast solid at the bottom;
(3) Step (2) was repeated 4 times to obtain 195g of clean yeast raw material (dry matter content is 32%);
(4) 200g of water was added to the clean yeast raw material, stirred evenly, and then a homogenizer was used to break the clean yeast raw material. The homogenizer pressure was set to 135MPa, the flow rate was set to 48ml/min, and the number of cycles was 5 times to obtain yeast wall-breaking liquid;
(5) The yeast wall-breaking liquid was separated, with the separator speed set to 5000 rpm, and the separation factor was 4472 at this time. Following separation for 10 minutes, the solid part was removed to obtain a liquid containing yeast cytoplasm material;
(6) A microfiltration membrane was used to microfilter the obtained liquid containing yeast cytoplasm material. The pore size of the microfiltration membrane was 0.2 µm. The medium was removed and small molecular substances therein by microfiltration to obtain 75g of concentrated yeast cytoplasm macromolecular substance (dry matter content is 20%);
(7) 225 g of pure water was added into the concentrated yeast cytoplasm macromolecular substance to obtain a yeast cytoplasm macromolecular substance dilution liquid;
(8) A microfiltration membrane was used to separate and concentrate the yeast cytoplasm macromolecular substance dilution liquid again to obtain 72 g of the first purified yeast cytoplasm macromolecular substance concentrate (dry matter content is 20%);
(9) Steps (7) and (8) were repeated 4 times to remove the medium and small molecular substances in the yeast cytoplasm macromolecular substance concentrate to obtain 68 g of the pure yeast cytoplasm macromolecular substance concentrate (dry matter content is 20%);
(10) The obtained pure yeast cytoplasm macromolecular substance concentrate was separated, and the separator speed was set at 6000 rpm. at this time, the separation factor was 6439. The solid matter was removed, and finally 66g of pure, colorless and odorless yeast cytoplasm macromolecular extract (dry matter content was 19.5%) with original biological activity was obtained.

### Yeast cytoplasm macromolecular extract detection:

Chroma determination: The light industry standard of the People's Republic of China QB/T 2789-2006 "General test method for cosmetics for color tristimulus value and color difference △E* determination" was adopted.

The measurement result: the average reading ΔE* of two sample preparation measurements was 5.2, which was almost pure white.

Odor determination: The sensory measurement was adopted, and the test result was that there was almost no special odor produced by yeast during the fermentation and cultivation process.

Determination of inorganic salts: Using a conductivity meter (Ray Magnetics desktop conductivity meter DDS-307A), the test result was 0.02S/m, and the salt content in the yeast cytoplasmic macromolecular extract was less than 100ppm.

### EXAMPLE 2

(1) Starting with 200g of wet yeast (dry matter content is 33%) just cultured, 600g of water was added, the separator was set to 4500 rpm, the separation factor was 3622, separated for 10 minutes, and the supernatant was removed to obtain the yeast solid at the bottom;
(2) 600g of water was added into the yeast solid, stirred evenly, the separator was set to 4500 rpm, the separation factor is 3622, separated for 10 minutes and the supernatant was removed to obtain the yeast solid at the bottom;
(3) Step (2) was repeated 4 times to obtain 196g of clean yeast raw material (dry matter content is 32%);
(4) 200g of water was added to the clean yeast raw material, stirred evenly, and then a homogenizer was used to break the clean yeast raw material. The homogenizer pressure was set to 135MPa, the flow rate was set to 48ml/min, and the number of cycles was 5 to obtain yeast wall-breaking liquid;
(5) The yeast cell wall liquid was separated, the separator speed set at 5000 rpm, and the separation factor was 4472. Following separation for 10 minutes, the solid part was removed to obtain a liquid containing yeast cytoplasm material;
(6) A microfiltration membrane to microfilter the obtained liquid containing yeast cytoplasm material was used, the pore size of the microfiltration membrane was 0.2 µm, and the medium and small molecular substances were removed therein by microfiltration to obtain 79g of concentrated yeast cytoplasm macromolecular substance (dry matter content is 20%);
(7) 237g of pure water was added to the concentrated yeast cytoplasm macromolecular substance to obtain a yeast cytoplasm macromolecular substance dilution liquid;
(8) A microfiltration membrane to separate and concentrate the yeast cytoplasm macromolecular substance dilution liquid was used again to obtain 75g of the first purified yeast cytoplasm macromolecular substance concentrated liquid (dry matter content is 20%);
(9) Steps (7) and (8) were repeated 4 times to completely remove the medium and small molecular substances in the yeast cytoplasm macromolecular substance concentrate to obtain 71g of pure yeast cytoplasm macromolecular substance concentrate (dry matter content is 20%);
(10) The obtained pure yeast cytoplasm macromolecular substance concentrate was separated, with the separator speed at 6000 rpm, and the separation factor was 6439 at this time, to remove the solid matter therein, and finally obtain 69g of pure, colorless and odorless yeast cytoplasm macromolecular extract (dry matter content is 19.5%) that retains the original biological activity.

### Detection of yeast cytoplasm macromolecular extract:

Chroma determination: The light industry standard of the People's Republic of China QB/T 2789-2006 "General test method for cosmetics determination of color tristimulus values and color difference ΔE*" was adopted. The test result: the average reading of the two sample preparation measurements ΔE* was 3.2, which was pure white.

Determination of odor: The sensory test was adopted, and the test result showed that there was no special odor produced by yeast during the fermentation and cultivation process.

Determination of inorganic salts: The conductivity meter (Ray-Magnetics desktop conductivity meter DDS-307A) was used, and the test result was 0.016S/m. The salt content in the yeast cytoplasm macromolecular extract was less than 80ppm.

### EXAMPLE 3

(1) 800g of water as added to 200g of waste yeast (dry matter content is 33%), the separator was set to 5000 rpm, with a separation factor of 4472, and separated for 10 minutes. The supernatant was removed to obtain the yeast solid at the bottom;
(2) 800g of water was added to the yeast solid, stirred evenly, the separator was set to 5000 rpm, with a separation factor of 4472, and separated for 10 minutes. The supernatant was removed to obtain the yeast solid at the bottom;
(3) Step (2) was repeated 4 times to obtain 194g of clean yeast raw material (dry matter content is 20%);
(4) 180g of water was added to the clean yeast raw material, stirred evenly, and then a homogenizer was used to break the clean yeast raw material. The homogenizer pressure was set to 145MPa, the flow rate was set to 48ml/min, and the number of cycles was 5 times to obtain yeast wall-breaking liquid;
(5) The yeast wall-breaking liquid was separated, with the separator speed set at 5500 rpm, and the separation factor at this time was 5411. Following 10 minutes of separation, the solid part was removed to obtain a liquid containing yeast cytoplasm material;
(6) A microfiltration membrane was used to microfilter the obtained liquid containing yeast cytoplasm material. The pore size of the microfiltration membrane was 0.3 µm. The medium and small molecular substances therein were removed by microfiltration to obtain 71g of concentrated yeast cytoplasm macromolecular substance (dry matter content is 20%);
(7) 365g of pure water was added into the concentrated yeast cytoplasm macromolecular substance to obtain a yeast cytoplasm macromolecular substance dilution liquid;
(8) A microfiltration membrane was used to separate and concentrate the yeast cytoplasm macromolecular substance dilution liquid again to obtain 69g of the first purified yeast cytoplasm macromolecular concentrate (dry matter content is 20%);
(9) Steps (7) and (8) were repeated 4 times to completely remove the medium and small molecular substances in the yeast cytoplasm macromolecular concentrate to obtain 64g of the pure yeast cytoplasm macromolecular concentrate (dry matter content is 20%);
(10) The obtained pure yeast cytoplasm macromolecular substance concentrate was separated, and the separator speed was set at 6000 rpm. The separation factor was 6439 at this time. The solid matter was removed, and finally 62g of pure, colorless and odorless yeast cytoplasm macromolecular extract (dry matter content was 19.5%) with original biological activity was obtained.

### Detection of yeast cytoplasm macromolecular extract:

Chroma measurement: The light industry standard of the People's Republic of China QB/T 2789-2006 "General test method for cosmetics determination of color tristimulus values and color difference ΔE*" was adopted. The measurement result: the average reading of the two sample preparation measurements ΔE* was 3.9, which was almost pure white.

Odor measurement: The sensory measurement was adopted, and the test result was that there was almost no special odor produced by yeast during the fermentation and cultivation process.

Inorganic salt determination: The conductivity meter (Raymag desktop conductivity meter DDS-307A) was used, and the test result was 0.0185S/m, and the salt content in the yeast cytoplasm macromolecular extract was less than 90ppm.

## Claims

1. A process for producing microbial cytoplasm macromolecular extract, comprising the steps of: (1) adding water to a used microbial raw material, preferably yeast, to obtain a microbial raw material dilution, followed by separation of the obtained microbial raw material dilution using a separator, and removing the liquid fraction to obtain a microbial solid; and
(2) repeating step (1) at least once to obtain a clean microbial raw material; and
(3) adding water to the clean microbial raw material to obtain a uniform clean microbial mixed liquid; and
(4) breaking the cell wall of the clean microbial mixed liquid to obtain a broken cell wall liquid of the microbial mixed liquid; and
(5) using a separator to separate the broken cell wall liquid of the microbial mixed liquid, separating and removing the solid part, to obtain a liquid containing microbial cytoplasm material; and
(6) using a microfiltration membrane to microfilter the obtained liquid containing microbial cytoplasm material, removing the medium and small molecular substances therein, to obtain a concentrated microbial cytoplasm macromolecular substance; and
(7) adding water into the concentrated microbial cytoplasm macromolecular substance to obtain a microbial cytoplasm macromolecular substance dilution; and
(8) separating and concentrating the microbial cytoplasm macromolecular substance dilution by a microfiltration membrane to obtain a first purified microbial cytoplasm macromolecular substance concentrate; and
(9) repeating steps (7) and (8) to remove medium and small molecular substances in the concentrated microbial cytoplasm macromolecular substances to obtain a pure microbial cytoplasm macromolecular substance concentrate; and
(10) separating the obtained pure microbial cytoplasm macromolecular substance concentrate by a high-speed centrifuge to remove solid substances therein to finally obtain a microbial cytoplasm macromolecular extract.

2. The process according to claim 1, wherein the microorganisms described in step (1) include unicellular eukaryotic microorganisms and/or unicellular prokaryotic microorganisms.

3. The process according to claim 1, wherein in step (1), the dry matter content of the microbial solid is not less than about 30% (w/w), calculated based on the weight percentage of dry matter contained in the microbial solid.

4. The process according to claim 1, wherein the separators described in steps (1), (5) and (10) can separate the solid and liquid phases in the separated liquid, and are selected from horizontal screw centrifuges, disc separators, plate and frame filter presses.

5. The process according to claim 1, wherein when adding water to the clean microbial raw material in step (3), the amount of water added is about 0.5-2 times (w/w) of the microbial raw material.

6. The process according to claim 1, wherein breaking the cell wall in step (4) is carried out at low temperature, optionally using a homogenizer to break the cell wall.

7. The process according to claim 1, wherein the medium and small molecular substances in step (6) contain all color and odor substances and inorganic salts in the obtained liquid containing microbial cytoplasm substances.

8. The process according to claim 1, wherein the overall process from step (1) to (10) does not comprise addition of enzymes or chemicals that destroy the biological activity of the substances in the microbial cytoplasm macromolecular extract, and the temperature is maintained at no more than about 55° C in the overall process.

9. The process according to claim 1, wherein the pore size of the microfiltration membrane is about 0.05-1 µm.

10. The process according to claim 1, wherein the microbial cytoplasm macromolecular extract in step (10) is in the form of a concentrated solution as the final product or is freeze-drying as a lyophilized powder as the final product.
